# EUROPEAN PATENT APPLICATION

(11) **EP 2 269 542 A1**
(43) Date of publication of application: **05.01.2011**
(21) Application number: 09425240.0
(22) Date of filing: 24.06.2009
(51) Int. Cl.: A61F 5/455

(54) **Hygienic device for urinals**

(71) Applicant: Balzano, Giovanni, 20138 Milano (IT)
(72) Inventor: Balzano, Giovanni, 20138 Milano (IT)
(74) Representative: Lunati & Mazzoni S.r.L.

(57) **Abstract**

There is described a hygienic device (1) for urinals, having a drain base, and comprising: an inlet (2), suitable to be gripped manually and to allow insertion of the male genital organ inside the inlet (2), a tubular duct (3) in fluid communication with the inlet (3), and an outlet (4) opposite the inlet (2), wherein the tubular duct (3) defines a distance between the inlet (2) and the outlet (4) such as to allow a user standing to place the outlet (4) in proximity of the drain base and wherein the hygienic device (1) degrades rapidly in water.

## Description

### HYGIENIC DEVICE FOR URINALS

The present invention concerns a hygienic device, usable in urinals, of the type specified in the preamble of the first claim.

As it is known, human males urinate while standing into toilet bowls, squat toilets or other urinals.

Said position allows the operation to be performed rapidly, but causes some drawbacks.

In fact, due to the height of the stream, when the urine comes into contact with the urinal this causes splashing which can spatter out of the urinal and soil the floor or clean portions of the urinal.

The drawback is particularly noticeable in toilet bowls, which are present substantially in all dwellings, offices and public places.

Toilet bowls in fact comprise a top rim for sitting on which must not be soiled.

In this situation the technical aim underlying the present invention is to devise a hygienic device for urinals capable of substantially overcoming the aforesaid drawbacks.

Within said technical aim, an important object of the invention is to achieve a hygienic device for urinals which prevents splashing during urination.

A further object of the invention is to devise a hygienic device for urinals which can be adapted to toilet bowls.

Another important object of the invention is to produce a simple and inexpensive hygienic device for urinals.

The technical aim and the objects specified are attained by a hygienic device for urinals according to the appended claim 1.

Preferred embodiments are highlighted in the dependent claims.

Further characteristics and advantages of the invention are clarified below in a detailed description of a preferred embodiment of the invention with reference to the attached drawings, wherein:

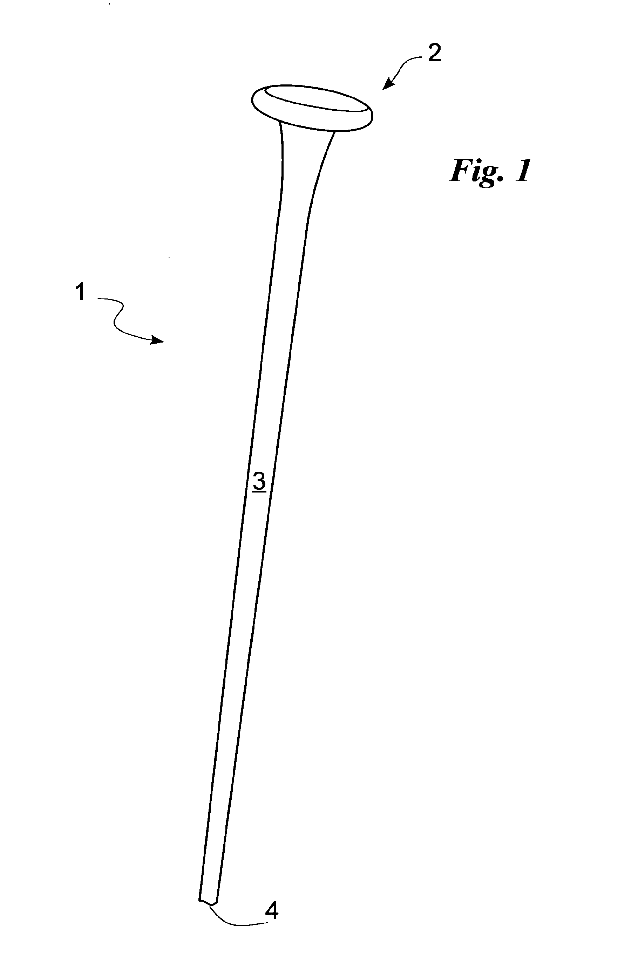

Figure. 1 shows the hygienic device according to the invention;
Figure. 2 shows a sagittal section of the hygienic device; and
Figure. 3 shows a plurality of hygienic devices in stacked position.

With reference to the aforesaid Figures, the hygienic device according to the invention is indicated as a whole with the number 1.

It can be used in urinals, in particular in toilet bowls, having a drain base into which urine is discharged. In particular, if the urinal is a toilet bowl, the drain base is the water present on the bottom thereof or simply the bottom without water or a lateral wall.

The hygienic device 1 comprises an inlet 2, a tubular duct 3, in fluid communication with the inlet 3, and an outlet 4 opposite the inlet 2.

The inlet 2 is suitable to be gripped manually and to allow insertion of the male genital organ inside the inlet 2. In particular, the inlet 2 is in one piece with the tubular duct 3 and is realized by an enlarged and curved portion of the tubular duct 3.

It is therefore suitable to be gripped with the index finger and thumb of only one hand and has an external diameter preferably of between 5 cm and 10 cm and is approximately truncated-cone shaped.

The inlet 2 can also comprise handles, suitable to facilitate gripping of the hygienic device 1.

The tubular duct 3 defines a distance between the inlet 2 and the outlet 4 such as to allow a user who is urinating while standing to place the outlet 4 in proximity of the drain base of the urinal and in particular of the toilet bowl.

The length of the tubular duct 3 is therefore preferably of between 0.3 m and 1.2 m and more preferably of between 0.5 m and 1 m.

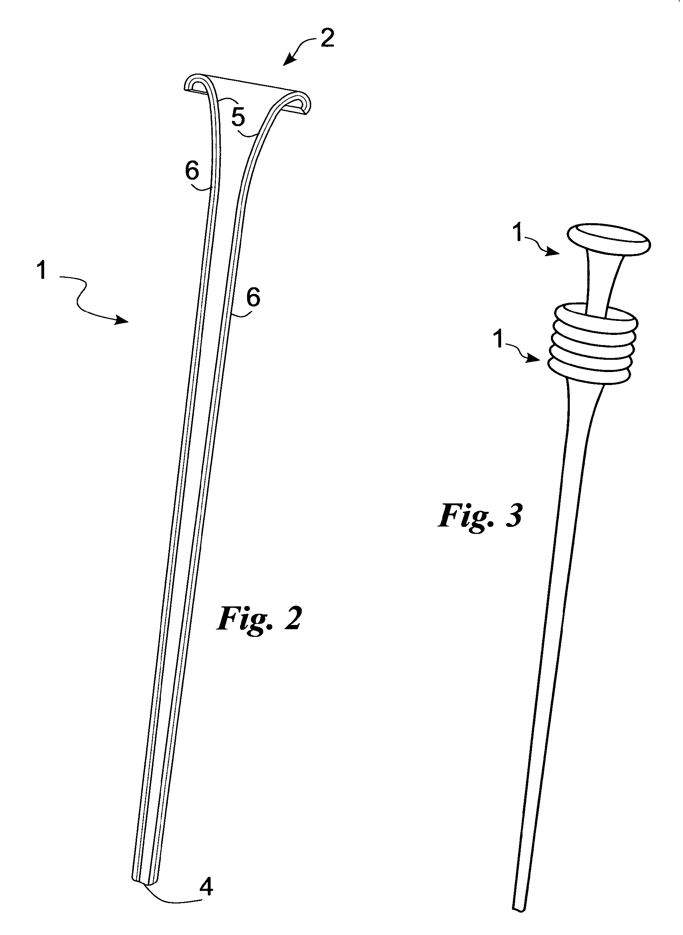

Moreover, it is conveniently slightly truncated-cone shaped in a manner suitable to allow stacking of a plurality of hygienic devices 1, as shown in Fig. 3.

The device also degrades rapidly in water.

Therefore, it can be abandoned inside the urinal without problems, in particular if realized by a toilet bowl.

The hygienic device 1 is, for this purpose, made substantially of paper and more preferably of toilet paper or a similar material.

In particular, it can be made of sheets produced with Starch or the like, Salt, Sugar and the like and in particular with rapidly biodegradable polymers. Moreover, it conveniently comprises an inner layer 5 and an outer layer 6. The inner layer 5 degrades more slowly than the outer layer 6 and is suitable not to degrade directly under the action of the stream of urine.

Operation of a hygienic device 1, the structure of which is described above, is the following.

The device 1 is gripped at the inlet 2 by a male user who requires to urinate. The genital organ is inserted, at least partly, inside the inlet 2 and the outlet 4 is placed in proximity of the bottom of the urinal, in particular in proximity of the water present in a toilet bowl.

During urination the urine passes through the tubular duct 3 and exits from the outlet 4. Therefore, this prevents splashes which can spatter out of the urinal. At the end of the operation it is sufficient for the user to drop the hygienic device 1 into the drain.

The invention achieves important advantages.

In fact, the device prevents any splashes outside the urinal and in particular outside the toilet bowl.

It is also simple and inexpensive, can be used with any type of urinal. Moreover, it is easy to dispose of, and in fact can merely be dropped into the drain.

Finally, the device 1 is stackable and therefore easy to store.

The invention is susceptible to modifications and variants falling within the inventive concept. All the details can be replaced by equivalent elements and the shapes and dimensions can be any.

## Claims

1. A hygienic device (1) for urinals, having a drain base, and comprising:
- an inlet (2), suitable to be gripped manually and to allow insertion of the male genital organ inside said inlet (2),
- a tubular duct (3) in fluid communication with said inlet (3) and
- an outlet (4) opposite said inlet (2),
- - **characterized in that**
- said tubular duct (3) defines a distance between said inlet (2) and said outlet (4) such as to allow a user who is standing to place said outlet (4) in proximity of said drain base and
- **in that** said hygienic device (1) degrades rapidly in water.

2. The hygienic device (1) according to claim 1, made substantially of paper.

3. The hygienic device (1) according to claim 2, made substantially of toilet paper.

4. The hygienic device (1) according to claim 1, made substantially of rapidly biodegradable polymers.

5. The hygienic device (1) according to claim 1, made substantially of starch.

6. The hygienic device (1) according to claim 1, made substantially of sheets of salt.

7. The hygienic device (1) according to claim 1, made substantially of sheets of sugar.

8. The hygienic device (1) according to one or more of the preceding claims, comprising an inner layer (5) and an outer layer (6), said inner layer (5) degrading more slowly than said outer layer (6).

9. The hygienic device (1) according to one or more of the preceding claims, wherein said tubular duct (3) defines a distance between said inlet (2) and said outlet (4) of between 0.3 and 1.2 m.

10. The hygienic device (1) according to claim 9, wherein said tubular duct (3) defines a distance between said inlet (2) and said outlet (4) of between 0.5 and 1 m.

11. The hygienic device (1) according to one or more of the preceding claims, wherein said inlet (2)) has an external diameter of between 5 cm and 10 cm.

12. The hygienic device (1) according to one or more of the preceding claims, wherein said inlet (2) is in one piece with said tubular duct (3) and is realized by an enlarged and curved portion of the tubular duct (3).

13. The hygienic device (1) according to one or more of the preceding claims, wherein said inlet (2) is approximately truncated-cone shaped.

14. The hygienic device (1) according to one or more of the preceding claims, wherein said tubular duct (3) is slightly truncated-cone shaped and said hygienic device (1) is stackable with other similar hygienic devices (1).
